# EUROPEAN PATENT SPECIFICATION

(11) **EP 4 126 806 B1**
(45) Date of publication and mention of the grant of the patent: **08.05.2024**
(21) Application number: 21712886.7
(22) Date of filing: 24.03.2021
(51) Int. Cl.: C07C 45/50, C07C 47/02

(54) **METHOD FOR THE HYDROFORMYLATION OF OLEFINS**
VERFAHREN ZUR HYDROFORMYLIERUNG VON OLEFINEN
PROCÉDÉ D'HYDROFORMYLATION D'OLÉFINES

(30) Priority: 03.04.2020 EP 20167893
(43) Date of publication of application: 08.02.2023
(73) Proprietor: BASF SE, 67056 Ludwigshafen am Rhein (DE)
(72) Inventor: PAPP, Rainer, 67056 Ludwigshafen (DE); HUETTEN, Frank, 67056 Ludwigshafen (DE); JUNGMANN, Dirk, 67056 Ludwigshafen (DE); RICHMOND, Edward, 67056 Ludwigshafen (DE)
(74) Representative: BASF IP Association
(86) International application number: PCT/EP2021/057526
(87) International publication number: WO 2021/197953

(56) References cited:
- US-A1- 2015 141 702

## Description

The present invention is defined in the appended claims and relates to a process for hydroformylation of olefins having 6 to 20 carbon atoms.

Hydroformylation also known as oxo process is an important large-scale industrial process for preparing aldehydes from olefins, carbon monoxide and hydrogen. These aldehydes can optionally be hydrogenated with hydrogen in the same operation or subsequently in a separate hydrogenation step, to produce the corresponding alcohols. Hydroformylation is carried out in the presence of catalysts which are homogeneously dissolved in the reaction medium. Catalysts used are generally the carbonyl complexes of metals of transition group VIII, in particular Co, Rh, Ir, Pd, Pt or Ru, which may be unmodified or modified with, for example, amine-containing or phosphine-containing ligands. A summarizing account of the processes practiced on a large scale in industry is found in J. Falbe, "New Syntheses with Carbon Monoxide", Springer Verlag 1980, p. 162 ff.

While short-chain olefins with up to 5 carbon atoms are currently predominantly hydroformylated using ligand-modified rhodium carbonyls as the catalyst, cobalt remains the dominant catalytically active central atom for longer-chained olefins. This is due, firstly, to the high catalytic activity of the cobalt carbonyl catalyst irrespective of the position of the olefinic double bonds, the branch structure and the purity of the olefin to be reacted. Secondly, the cobalt catalyst can be separated off from the hydroformylation products and recycled into the hydroformylation reaction relatively easily. Additionally, catalyst losses during working up can be tolerated more easily owing to the lower price of cobalt.

In one customary process for separating off and recycling the cobalt catalyst, the organic phase of the reactor affluent is freed of cobalt carbonyl complexes by treatment with oxygen or air in the presence of weakly acidic water (cf. DE-AS 24 04 855). In the treatment, the cobalt catalyst is destroyed by oxidation and the central atom is formally converted from the oxidation state -1 to +2 and can then be removed by extraction with the aqueous solution (decobalting). The catalyst complex required for hydroformylation can be re-formed from the cobalt(II) salt solution by reaction with carbon monoxide and hydrogen (carbonyl formation). The re-formed cobalt catalyst is then extracted from the aqueous phase with an organic phase, preferably the olefin to be hydroformylated (catalyst extraction). Besides the olefin, the reaction products and by-products of the hydroformylation can also be used for catalyst extraction. The olefins loaded with the cobalt catalyst are then hydroformylated in a reactor at elevated pressure and elevated temperature (olefin hydroformylation).

The reaction output is customarily withdrawn at the top of the reactor, in particular when a vertical tubular reactor is used. Particularly with higher olefins having 8 carbon atoms or more, the aqueous phase supplied to the reaction zone and necessary to achieve a sufficient catalyst concentration in the reaction zone is not entirely discharged in dissolved or suspended form with the reaction mixture removed from the top. Therefore, reaction output is preferably removed not only at the top, but also from the bottom space.

The uncontrolled or insufficiently controlled withdrawal of aqueous phase from the bottom space leads to operational disruption. An excess of aqueous phase can lead to a decrease in conversion. A deficiency of aqueous phase can lead to local temperature spikes which cause decomposition of the cobalt catalyst.

In the prior art, the amount of reaction output withdrawn from the bottom space is determined in accordance with the level of the aqueous bottom phase in the reactor.

EP 1 204 624 B1 discloses a continuous process for hydroformylation of olefins having 6 to 20 carbon atoms wherein a cobalt catalyst-containing aqueous phase is brought into intimate contact with olefins, hydrogen and carbon monoxide in at least one reaction zone (e.g. in a vertical tubular reactor). Reaction output can be withdrawn not only at the top of the reactor but also from the bottom space of the reactor. The withdrawal of reaction output from the bottom space of the reactor is preferably phase regulated.

EP 1 279 658 B1 discloses a process for hydroformylation of olefins having 5 to 24 carbon atoms to produce the corresponding aldehydes and/or alcohols having 6 to 25 carbon atoms in the presence of unmodified cobalt catalysts in a single-step process wherein the aqueous bottom phase is thoroughly mixed in the reactor with the organic phase, the concentration of cobalt compounds in the aqueous bottom phase is in the range from 0.4 to 1.7% by weight and the level of the aqueous bottom phase is kept constant at a steady state.

EP 3 071 542 B1 and US 2015/141 702 A1 discloses a process for hydroformylation of olefins having 6 to 20 carbon atoms to produce the corresponding hydroformylation products in the presence of a cobalt catalyst in the presence of an aqueous phase with thorough mixing in the reactor wherein an aqueous phase-containing stream is withdrawn from the bottom of the reactor and wherein the flow rate of this aqueous phase-containing stream is controlled in accordance with the temperature measured at a point at the bottom of the reactor or in a line leading out of the bottom of the reactor.

However, the level of the aqueous bottom phase often cannot be determined accurately under the conditions prevailing in the reactor.

This was especially observed in larger reactors when withdrawal of the aqueous phase was controlled in accordance with the temperature measured at a point at the bottom of the reactor or in a line leading out of the bottom of the reactor as disclosed in EP 3 071 542 B1.

Radiometric measurement of the level of the aqueous bottom phase is imprecise. The γ-radiation is attenuated by passage through the 30 cm-thick steel walls. Additionally, the calibration of the radiometric measurement is difficult since the level of the aqueous bottom phase in the reactor is, as a rule, not known precisely even at calibration.

Further, the maintenance of apparatuses for determining the level of the aqueous bottom phase is very costly and inconvenient when they are disposed inside the reactor and therefore only accessible at great cost and inconvenience by interrupting the process.

The imprecise determination of the level of the aqueous bottom phase leads to an insufficiently precisely controllable withdrawal of aqueous phase from the bottom space and thereby to operational disruptions that ultimately also lower the yield of crude hydroformylation product. Therefore, it was an object of the present invention to provide an improved process for the hydroformylation of olefins having 6 to 20 carbon atoms which delivers an increased yield of crude hydroformylation product as a result of stable sustained operation.

It was found out that with increasing reactor size specific heat loss over the reactor surface decreases. Thus, the temperature difference between the temperature measured within the reactor in a zone of vigorous mixing and the temperature measured at the bottom of the reactor or in a line leading out of the bottom of the reactor becomes insignificant and precise control by means of temperature differences is no longer possible.

Thus, the improved process should allow to precisely control the withdrawal of an aqueous phase containing-stream independent of temperature differences in the reactor in a zone of vigorous mixing and the temperature measured at the bottom of the reactor or in a line leading out of the bottom of the reactor.

The objective is achieved by a process for hydroformylation of olefins having 6 to 20 carbon atoms in the presence of a cobalt catalyst in the presence of an aqueous phase with thorough mixing in a reactor wherein a hydroformylation products-containing first stream is withdrawn at the top of the reactor and an aqueous phase-containing second stream is withdrawn from the bottom of the reactor via at least one line leading out of the bottom of the reactor, which process comprises controlling one or more mass flow parameters of the second stream in accordance with the density of the second stream, which is preferably measured in the line leading out of the bottom of the reactor.

The process can be continuous, semi-continuous or batch-wise. A continuous or semi-continuous process is preferred.

A mass flow parameter is the mass flow, the mass flow rate or any other parameter determined on the basis of the mass flow and/or mass flow rate. Preferably at least one mass flow parameter is the mass flow or the mass flow rate.

As the aqueous phase separates from the organic phase and separates out in the bottom space of the reactor, it no longer undergoes mixing with olefins to be hydroformylated. The reaction rate in the bottom space of the reactor falls and the separated-out aqueous phase slowly cools down. As stated above, this cooling may be very limited where the reactor's volume-to-surface ratio and any insulation reduce dissipation of the heat generated in the reactor and thus decrease any temperature differences in the reactor. Controlling the withdrawal of the bottom stream from a large reactor based on the temperature difference between reactor bottom and reaction zone therefore necessitates an amount of non-reacting aqueous phase at the bottom that is large enough to cool down enough for developing a temperature difference to the reaction zone that is significant enough for use as input for the control loop. A large amount of non-reacting aqueous phase in the reactor, however, reduces reactor volume utilization and productivity and thus counteracts any economy of scale.

It was found that an aqueous phase-containing stream can be withdrawn from the bottom space of the reactor under density control.

The basis for this finding is that when the stream drawn from the bottom of the reactor is greater than the amount of water settling at the current point in time, organic phase, too, is withdrawn from the bottom of the reactor, so that the aqueous stream withdrawn from the bottom of the reactor also contains an amount of dispersed organic phase. The density of this stream depends on the ratio of the two phases. The precise dependence may be more complex than linear but can be easily determined and the desired set point for controlling the withdrawal can be determined according to the specific conditions of the reactor and the plant. In contrast to controlling the withdrawal based on a temperature difference in the reactor, the inventive method allows removing the water from the reactor to any desired extent. Said in a very basic manner, if the withdrawn liquid has the density of the pure aqueous phase, more can be withdrawn to increase reactor productivity, and if the withdrawn liquid has the density of pure organic phase, too much liquid is withdrawn. In real life operation of these reactors of the stirred-tank type, the density of the stream withdrawn from the bottom will be between these extremes of pure aqueous or pure organic phase and vary depending on their relative amounts in that stream. One advantage of the present invention is that the desired amount of aqueous phase can be removed while avoiding removing too much organic phase. The amount of organic phase withdrawn at the bottom of the reactor is insignificant in comparison to the overall organic outlet of the reactor. In a preferred embodiment, any continuous aqueous phase is removed from the reactor and only dispersed aqueous phase remains.

The inventive method allows withdrawing exactly the desired amount of aqueous phase. This prevents the aqueous phase rising to too high a level. Thus, unintended succumbing of the hydroformylation reaction can be avoided and continuous operation can be achieved. In other words, the reaction can be stabilized. It furthermore also minimizes the amount of water in the reactor as no continuous water-phase is present in the reactor. This leads to a maximization of the available reaction volume and therefore a maximization of conversion in the reactor.

Advantageously, the density of the second stream (second stream is used as synonym to the aqueous phase-containing stream) can be accurately determined independent of any temperature differences between any points in the reactor. Thus, control of the at least one mass flow parameter of the second stream in accordance with the density of the second stream provides efficient removal of the of the aqueous phase in the reactor independent on the reactor size. Furthermore, the fraction of the aqueous phase in the reactor can be minimized by this approach and therefore the conversion in the reactor can be increased as a higher residence time of the organic phase is realized.

Preferably, controlling of the one or more mass flow parameters of the second stream in accordance with the density of the second stream comprises measuring the density of the second stream to determine one or more controlled variables on basis of the measured density, comparing the one or more controlled variables with one or more reference values to determine one or more controlling quantities and using the one or more controlling quantities to determine one or more actuating signals to control the one or more mass flow parameters of the second stream. It is even more preferred that control of the one or more mass flow parameters of the second stream in accordance with the density of the second stream is performed by means of a closed loop control system comprising the aforementioned control steps.

The density of the second stream can be measured by any means and at any point suitable for density measurements. It is preferred that the density of the second stream is measured in the line leading out of the bottom of the reactor.

In case the density of the second stream is measured in the line leading out of the bottom of the reactor, the density is preferably measured by one or more Coriolis mass flow meters.

Coriolis mass flow meters are commercially available, and their mode of operation is known to the person skilled in the art. When measuring the density of the second stream by means of one or more Coriolis mass flow meters in the line leading out of the bottom of the reactor, the person skilled in the art will be guided by common general knowledge how many Coriolis mass flow meters to use and how to install them in connection with the line leading out of the bottom of the reactor.

Of course, the density of the individual phases and therefore of the two-phased stream also depend on temperature. Determining the temperature-dependency of the density of the individual phases can easily be done by determining the density at different temperatures. The density of the mixture at those temperatures can the easily be calculated as the weight-average of the density of the two phases. This enables calibrating the density sensor at the reactor outlet to the temperature measured at the reactor outlet. It may be preferable, as in other cases of controlling a valve or the like on the basis of a temperature-dependent value, to use the temperature-corrected value as basis for controlling. In order to keep the ratio of the two phases in the stream taken from the reactor constant, it therefore may be preferable to use the temperature-corrected density as basis for controlling the total amount of that stream instead of the density at the actually measured temperature of the stream. The reference point for the temperature compensation may be chosen arbitrarily, however it is preferable to select a temperature close to the operating temperature of the reactor. Temperature-corrected density sensors, in particular temperature-corrected Coriolis density sensors, are commercially available, alternatively, the temperature-dependency of the density of the two-phase mixture may be stored in the process control system. In other words, the density measured at the reactor outlet may be shown to the operator as "Density at x °C", where x is the chosen reference temperature in °C, and the control logic will keep that value and thereby the ratio of the two phases constant.

On basis of the measured density of the second stream the one or more control variables can be determined by mathematical transformation of the measured density. The mathematical models and/or algorithms to be applied for this transformation are known to the person skilled in the art. In the simplest case, one control variable is the measured density of the second stream.

The one or more reference values can be static and/or dynamic values. In case of static values, the one or more reference values are predetermined values which may be changed from time to time. In case of dynamic values, the one or more reference values are controlled and/or optimized to increase the yield of crude hydroformylation product in accordance with one or more variables determined from the respective reaction plant and/or other reaction plants in connection with the respective reaction plant, like reaction temperature, yield of crude hydroformylation products, feed composition and/or catalyst load. Control and/or optimization of the one or more reference values is carried out in a control unit. Preferably the control unit, e.g. implemented in a central computing device / distributed control system or provided by means of cloud computing, is configured to execute an analysis algorithm based on the results of a machine-learning algorithm. The machine learning algorithm preferably comprises decision trees, naive bayes classifications, nearest neighbors, neural networks, convolutional neural networks, generative adversarial networks, support vector machines, linear regression, logistic regression, random forest and/or gradient boosting algorithms. Preferably, the machine-learning algorithm is organized to process an input having a high dimensionality into an output of a much lower dimensionality. Such a machine-learning algorithm is termed "intelligent" because it is capable of being "trained". The algorithm may be trained using records of training data. A record of training data comprises training input data and corresponding training output data. The training output data of a record of training data is the result that is expected to be produced by the machine-learning algorithm when being given the training input data of the same record of training data as input. The deviation between this expected result and the actual result produced by the algorithm is observed and rated by means of a "loss function". This loss function is used as a feedback for adjusting the parameters of the internal processing chain of the machine-learning algorithm. For example, the parameters may be adjusted with the optimization goal of minimizing the values of the loss function that result when all training input data is fed into the machine-learning algorithm and the outcome is compared with the corresponding training output data. The result of this training is that given a relatively small number of records of training data as "ground truth", the machine-learning algorithm is enabled to perform its job well for a number of records of input data that are higher by many orders of magnitude. It is preferred that the machine-learning algorithm is trained based on the input data.

In the simplest case one reference value is a predetermined reference value for the density of the second stream.

Preferably the one or more reference values and the one or more controlled variables are input parameters to one or more controllers and the one or more controlling quantities are determined on basis of the input parameters by applying mathematical models and/or algorithms. The mathematical models and/or algorithms to be applied in the one or more controllers to determine the one or more controlling quantities are known to the person skilled in the art or can be derived on basis of simple tests and general common knowledge. Suitable controllers are for example e.g. PID controllers, Model Predictive Controllers, adaptive controllers, non-linear controllers, fuzzy controllers or state space controllers.

On basis of the one or more controlling quantities one or more actuating signals are determined to control the one or more mass flow parameters of the second stream. The one or more controlling quantities are input parameters to one or more actuators. An actuator is for examples a valve with an actuator. The actuator can be operated pneumatically, hydraulically, electronically, electromagnetically, manually or by any mixed form of the aforementioned. In case the actuator is a valve with an actuator the actuating signal is the opening state of the valve.

In a simple case, if the measured density of the second stream is above a predefined reference value for the density of the second stream, the mass flow and/or the mass flow rate of the second stream is increased. In case the measured density of the second stream is below a predefined reference value for the density of the second stream, the mass flow and/or the mass rate of the second stream is decreased.

Suitable pressure-resistant reactors for carrying out the process according to the invention are known to the person skilled in the art. These include the generally customary reactors for gas-liquid reactions such as e.g. tubular reactors, stirred tanks, gas circulation reactors, bubble columns, loop reactors etc. which, optionally, can be further subdivided by internals. Examples of suitable reactors are a loop reactor, a vertical high-pressure bubble column reactor optionally equipped with coaxial tube-shaped internals, or a vertical tubular reactor. Preferably, the reactor is a vertical bubble column reactor or a vertical tubular reactor.

The temperature in the reactor is generally from 100 to 250 °C, in particular from 145 to 200 °C. The prevailing pressure in the reactor is preferably from 100 to 400 bar abs, particularly from 200 to 300 bar abs.

Part of the reaction mixture contained in the reactor can be discharged in the upper region of the reactor and fed back into the lower region of the reactor in order to vary the thorough mixing. By way of example, the discharged reaction mixture can be passed through a pump and then combined with the starting materials and the starting materials combined with the discharged reaction mixture can be fed into the reactor.

The term "olefins" herein denotes one olefin as well as mixtures of different, e.g. isomeric, olefins. Olefins having 6 to 20 carbon atoms may be hydroformylated by the process according to the invention. The process according to the invention is particularly suitable for the hydroformylation of isomeric olefin mixtures which are prepared by oligomerisation of lower olefins, such as propene and butenes. Typical oligomers useful as olefins for the present process include, inter alia, di-, tri- and tetrapropene, di-, tri- and tetrabutene and co-oligomers of propenes and butenes. The oligomers of butenes are obtainable on a large industrial scale via known oligomerisation processes, e.g. via the Octol process of Hüls and the Dimersol process of IFP. Preference in given to using oligomers based on so-called raffinate 2, a stream containing essentially linear butenes, n- and iso- butane, and not more than 5 % by weight of isobutene, preferably not more than 3 % by weight of isobutene. Linear long-chain terminal olefins, obtainable e.g. by the SHOP process or Ziegler process, or linear long-chain internal olefins can also be hydroformylated according to the process according to the invention.

The hydroformylation is carried out in the presence of hydrogen and carbon monoxide. The two gases are customarily used in the form of a mixture; so-called synthesis gas. The composition of the synthesis gas used in the process according to the invention can vary between wide limits. The molar ratio of carbon monoxide to hydrogen is, as a rule, about 10 : 1 to 1 : 10, in particular 2.5 : 1 to 1 : 2.5. A preferable ratio is about 40 : 60 (CO : H₂).

The catalyst extraction and olefin hydroformylation are carried out in one step in the reactor in the process according to the invention. The carbonyl formation can be carried out simultaneously or in an optional upstream catalyst formation step (precarbonylation).

In the optional catalyst formation step (precarbonylation), an aqueous cobalt(II) salt solution is brought into intimate contact with carbon monoxide and hydrogen to form the cobalt catalyst. This is carried out in the presence or absence of the olefins to be hydroformylated, preferably at temperatures of 50 to 200 °C, in particular 60 to 140 °C and under pressures of 100 to 400 bar abs, in particular 200 to 300 bar abs. The catalyst formation is preferably carried out in apparatus customary for gas-liquid reactions such as stirred tanks equipped with a sparging stirrer, bubble columns or trickle-bed columns. Advantageously, activated charcoal, zeolites or basic ion-exchangers, loaded with cobalt carbonyl, are used in the catalyst formation step as described in DE-OS 21 39 630. The cobalt(II) salts-containing- and cobalt catalyst-containing aqueous solution obtained in the catalyst formation step is then fed into the reactor together with the olefins to be hydroformylated and, optionally, any organic solvents used, as well as hydrogen and carbon monoxide.

The carbonyl formation is particularly advantageously carried out in the upstream catalyst formation step if linear, relatively long-chain terminal olefins are hydroformylated to form predominantly straight-chain aldehydes/alcohols. In order to minimize the formation of undesired branched hydroformylation products, as a rule, lower reaction temperatures are then employed in the reactor. However, under the conditions then employed in the reactor, the active cobalt catalyst is not formed at a sufficient rate.

In many cases, the lower technical requirements make it preferable for the carbonyl formation, catalyst extraction and olefin hydroformylation to be carried out in association with one another in the same reactor. In a preferred embodiment of the process, the formation of the cobalt catalyst, the extraction of the cobalt catalyst into the organic phase and the hydroformylation of the olefins are carried out in association with one another in the same reactor by bringing the aqueous cobalt(II) salt solution, the olefins and, optionally, organic solvents into intimate contact with one another in the reaction zone under hydroformylation conditions.

Irrespective of whether the carbonyl formation is or is not carried out in an upstream catalyst formation step, the starting materials are preferably introduced into the reactor such that good mixing of the phases results and as high as possible an exchange area between the phases is generated. The metering apparatuses known to the person skilled in the art, such as e.g. packed turbulence tubes or mixing nozzles for multiphase systems, can be used for this. The thorough mixing is particularly preferably carried out with a mixing nozzle to maintain a turbulent flow in the reactor. Thorough mixing is carried out in this way in a suitable embodiment by the aqueous cobalt(II) salt solution, olefins, carbon monoxide and hydrogen being simultaneously introduced into the reactor in a circulation system by means of a mixing nozzle as described in DE-AS 12 05 514 and 19 38 102. The optionally used organic solvent is either present in the reactor or is introduced into the reactor simultaneously with the other starting materials, in particular by means of a mixing nozzle.

Optionally usable organic solvents are inert hydrocarbons such as paraffin fractions, aromatic hydrocarbons such as benzene, toluene or xylene, or an aldehyde and/or alcohol; particularly, however, the hydroformylation product of the olefin used. High-boiling by-products of the hydroformylation can further be used as solvent. The use of a solvent can be advantageous in the hydroformylation of long-chain olefins to form long-chain aldehydes e.g. to lower viscosity.

The first stream, withdrawn at the top of the reactor, in addition to hydroformylation products, comprises unreacted olefins and significant amounts of aqueous phase. The second stream, withdrawn from the bottom of the reactor, can contain, besides the aqueous phase, significant amounts of partially reacted organic phase. Preferably, the second stream withdrawn from the bottom of the reactor contains 10 to 80 % by volume of aqueous phase.

The described reactor has a so-called stirred tank characteristic; this is a very thoroughly mixed reactor. In such a reactor, only partial conversion is possible with a limited reactor volume. It is therefore preferable, for the purposes of achieving as high a yield as possible of desired product, to continue the hydroformylation and ideally complete the hydroformylation, in a post-reactor. In a preferred embodiment, the first stream and the second stream are fed into a post-reactor.

The temperature in the post-reactor is generally from 100 to 250 °C, in particular from 145 to 200 °C. The prevailing pressure in the post-reactor is preferably in the region from 100 to 400 bar abs, in particular 200 to 300 bar abs. An even transport of material from the reactor to the post-reactor is preferably effected by the maintenance of a constant differential pressure of a few bar between the reactor and the post-reactor, with the post-reactor having a lower pressure. The differential pressure is preferably 2 to 5 bar. The differential pressure allows convenient control of the discharges from the first reactor. The differential pressure can be maintained by means of an adjustable valve or a diaphragm.

Preferably, at the post-reactor, too, a hydroformylation products-containing first stream is withdrawn at the top and an aqueous phase-containing second stream is withdrawn from the bottom. Preferably, too the second stream withdrawn from the bottom of the post-reactor is withdrawn under density control. Thus, in one preferred embodiment, the one or more mass flow parameter of the second stream withdrawn from the post-reactor is controlled in accordance with the density of the second stream, which is preferably measured in the line leading out of the bottom of the post-reactor.

The one or more mass flow parameters of the second stream withdrawn from the post-reactor are preferably controlled correspondingly by the same means as the one or more mass flow parameters of the second stream withdrawn from the first reactor. Embodiments which are preferred for the control of the one or more mass flow parameters of the second stream withdrawn from the reactor are also preferred embodiments for the control of the one or more mass flow parameters of the second stream withdrawn from the post-reactor.

Advantageously, a differential pressure between the post-reactor and the one or more discharge lines is set so that the discharges can be well controlled. This can be affected by means of pressure regulator like an adjustable valve or a diaphragm. This differential pressure is preferably also from 2 to 5 bar.

For work-up, the streams withdrawn from the reactor and if a post reactor is employed, the streams withdrawn from the post-reactor are decompressed to an intermediate pressure of 10 to 80 bar abs , preferably to 10 to 50 bar abs. Preferably the streams are combined either before, during or after decompression.

The decompressed streams are subjected in the presence of an aqueous cobalt (II) solution to oxygen treatment, wherein the cobalt catalyst decomposes to form cobalt(II) salts which are then extracted into the aqueous phase and the phases are then separated (decobalting step).

Preferably, oxygen is provided by supplying air. The treatment is preferably carried out at temperatures of 90 to 130°C.

The aqueous cobalt(lI) solution is preferably weakly acidic with a pH of 3 to 5, preferably 3.5 to 4.5. The aqueous cobalt (II) solution can be a fresh aqueous cobalt (II) solution to replenish cobalt(II) losses and/or part of the before separated aqueous cobalt(II) solution is recycled.

The treatment can be carried out in a packed (e.g. with Raschig rings) pressure vessel in which as high as possible an exchange area between the phases is generated, or else by decompression in an empty pressure vessel.

Separation of the aqueous phase from the organic phase is preferably carried out in a phase separation vessel. Suitable vessels or apparatuses for phase separation are for examples disclosed in Ullmann's Encyclopedia of Industrial Chemistry, Liquid-Liquid Extraction, DOI: 10.1002/14356007.b03_06.pub2. Mixer settler apparatuses are preferred as phase separation vessels.

The organic phase remaining after separating off the aqueous phase can then be appropriately worked up, e.g. distilled and/or hydrogenated.

The aqueous phase (cobalt(II) salt solution) is fed into the reactor or into the catalyst formation step or, alternatively, into the decobalting step.

The water content in the reactor, if a post reactor is used in the post reactor and in the organic aqueous phase separation is preferably controlled by means of dynamic decoupling to avoid oscillating operation conditions. The principle of dynamic decoupling, e.g. by complete dynamic decoupling by dynamic state feedback is well known to the person skilled in the art, especially in the field of automation technology.

Preferably, a distributed control system with PID controllers is used to control the one or more mass flow parameters of the second stream withdrawn from the reactor and the one or more mass flow parameters of the aqueous phase withdrawn from the phase separation, and if a post-reactor is used, to control the one or more mass flow parameters of the second stream withdrawn from the post-reactor. To avoid oscillating operation conditions in such a case by means of dynamic decoupling, one set of controllers is tuned to act slow in comparison to the other controllers. This is equivalent to a long time to steady state after a step change of the set-point of said controller. One set of controllers refers to the controllers used to control either a) the one or more mass flow parameters of the second stream withdrawn from the reactor, b) if a post-reactor is used, the one or more mass flow parameters of the second stream withdrawn from the post reactor, or c) the one or more mass flow parameters, of the aqueous phase withdrawn from the phase separation, for example the mass flow and/or the mass flow rate of the aqueous phase withdrawn from the phase separation vessel.

The avoidance of oscillating operation conditions has the advantage that for example the life time of the valves is increased as the cumulative travel of the valve stem commanded by control actions is reduced (lower valve travel index), the addition of fresh water is reduced and/or the loss of catalyst is reduced.

The process according to the invention is illustrated in more detail by the accompanying figure.

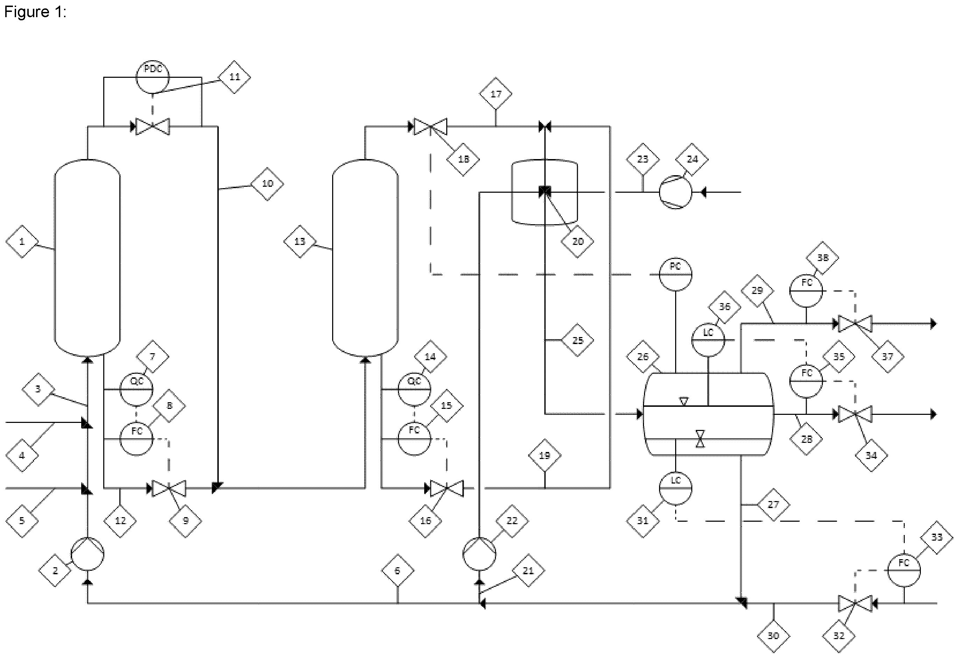

Figure 1 shows a schematic view of a plant for carrying out the process. The plant comprises a reactor 1 and a post-reactor 13, in which both the flow rate of the stream withdrawn from bottom of the reactor and the flow rate of the stream withdrawn from the bottom of the post-reactor are controlled in accordance with the invention.

As shown in figure 1, oxogas 4, olefin 5 and aqueous cobalt(II) salt solution 6 are introduced into a reactor 1 by means of a pump 2 through nozzle 3. The density of the stream withdrawn via the line leading out of the bottom of the reactor 12 is measured by means of Coriolis mass flow meter and used for control 7. The controller output of the density controller is supplied to the mass flow control unit 8 as a set point. The measured density is compared with a predefined reference value for the density. As soon as the measured density exceeds the predefined reference value, a signal to increase the mass flow is sent to the control unit 8 and the flow controller opens the valve 9. In the same manner as soon as the measured density falls below a predefined reference value for the density, a signal to close the valve 9 emanates from the control unit 8.

A hydroformylation products containing first stream is withdrawn at the top of the reactor 1 by means of line 10 and passed through a differential pressure regulator 11. Provided that valve 9 is open to some extent, an aqueous phase-containing second stream is withdrawn from the bottom of the reactor and passed by means of line 12 and merged with line 10.

The combined streams withdrawn at the top and from the bottom of the reactor are introduced into a post-reactor 13. The density of the stream withdrawn via the line 19 leading out of the bottom of the post-reactor is measured by means of a Coriolis mass flow meter and used for controller 14. The controller output of the density controller is derived by a comparison of a predefined reference value for the density and the measured density. It is used as set-point of the mass flow controller 15. As soon as the measured density exceeds the predefined reference value, a signal to increase the mass flow is sent to the control unit 15 and the flow controller opens valve 16. In the same manner as soon as the measured density falls below a predefined reference value for the density, a signal to close valve 16 emanates from the control unit 15. A hydroformylation products containing first stream is withdrawn at the top of post-reactor 13 by means of line 17 and is passed through control valve 18. Provided that valve 16 is open to some extent, an aqueous phase-containing second stream is withdrawn from the bottom of the post-reactor and combined with the stream from line 17.

The combined streams withdrawn at the top by line 17 and from the bottom of the post-reactor by line 19 are introduced into a hot-decompression vessel 20, which has a lower prevailing pressure than the reactor or the post-reactor and in which they are treated with air supplied by means of line 23 and compressor 24 in the presence of cobalt(II) salt solution which is supplied by means of line 21 and pump 22.

The mixture obtained in the hot-decompression vessel 20 is passed into the phase separator 26 by means of line 25 and an aqueous phase is discharged from the phase separator by means of line 27. The crude hydroformylation product is discharged from the phase separator by means of line 28. Offgas is discharged from the phase separator by means of line 29. The level of the liquid-liquid interface in the phase separator 26 is measured and controlled by the level controller 31. The controller output is used to manipulate the set-point of the water mass flow controller 33. The water mass flow is manipulated by changing the stem position of the valve 32 in the water line 30. The level of the gas-liquid interface in the phase separator 26 is measured and controlled by the level controller 36. The controller output is used to manipulate the set-point of the organic mass flow controller 35. The organic mass flow is manipulated by changing the stem position of the valve 34 in the product line 28. The off-gas mass flow is measured and controlled by the flow controller 38 by manipulation of the stem position of the valve 37 in the off-gas line 29.

### Examples

The examples do not limit the invention.

### Example 1

The following parameters were set in a continuous process carried out in a plant according to figure 1.

| | |
|---|---|
| oxogas (CO:H₂ = 40:60) (4) | 3 300 kg/h |
| isooctene (5) | 10 000 kg/h |
| aqueous cobalt(II) formate solution (6), ca. 1.2 % by weight of cobalt | 1 100 kg/h |
| reaction temperature in the reactor (1) | 187 °C |
| set-point of the density controller 7 | 0.998 g/cm³ |
| differential pressure in line (10) set by means of pressure regulator (controlled valve) (11) | 4 bar |
| reaction temperature in the post-reactor (13) | 187 °C |
| set-point of the density controller 14 | 0.998 g/cm³ |
| pressure of the phase-separator (26) set by means of pressure regulating control valve (18) | 19 bar |
| aqueous cobalt(ll) salt solution (6) | 9 000 kg/h |
| air (23) | 50 kg/h |
| offgas (29) | 500 kg/h |

The yield of crude hydroformylation product (28) was 12 400 kg/h.

### Example 2

A laboratory miniplant system was used for continuous hydroformylation of octene. The olefin was fed to a first stirred reactor using a high-pressure pump. The synthesis gas was taken from a high-pressure pipeline. The reactor was operated at a pressure of around 300 bar.

As catalyst precursor, an aqueous solution containing 1.8 wt.-% of cobalt formiate was fed to the reactor by a high-pressure pump. (Under those conditions, the carbon monoxide present in the reactor acts as ligand for the catalyst, the catalyst forms in-situ in the reactor and moves from the water phase to the organic phase.)

The reactor discharge is fed to a lower pressure vessel and air is added to oxidise and inactivate the catalyst and to remove it from the organic phase to the water phase. The discharge of that oxidation vessel is fed to a phase separator. The feed flow to the phase separator was 431-451 g/h. Thereof, approximately 50 g/h was water fed with the catalyst solution to the reactor. The phase separator was neither heated nor thermally insulated, consequently its contents were of uniform temperature. In the phase separator, the water phase settled to the bottom and was discharged. In this miniplant, the water phase was discarded and not recycled to the reactor.

A Coriolis mass flow meter measuring and reporting mass flow, temperature and density of the discharged stream was installed in the pipe used to discharge the water from the phase separator. The density measurement was used as process variable input to a density controller, which manipulated a valve installed in the pipe used to discharge the water phase. The density set point of the controller was chosen such that the density of the discharged stream at the given temperature of 40-50 °C is in the two-phase region. This effectively ensured that all water phase was discharged from the phase separator and no liquid level measurement was needed.

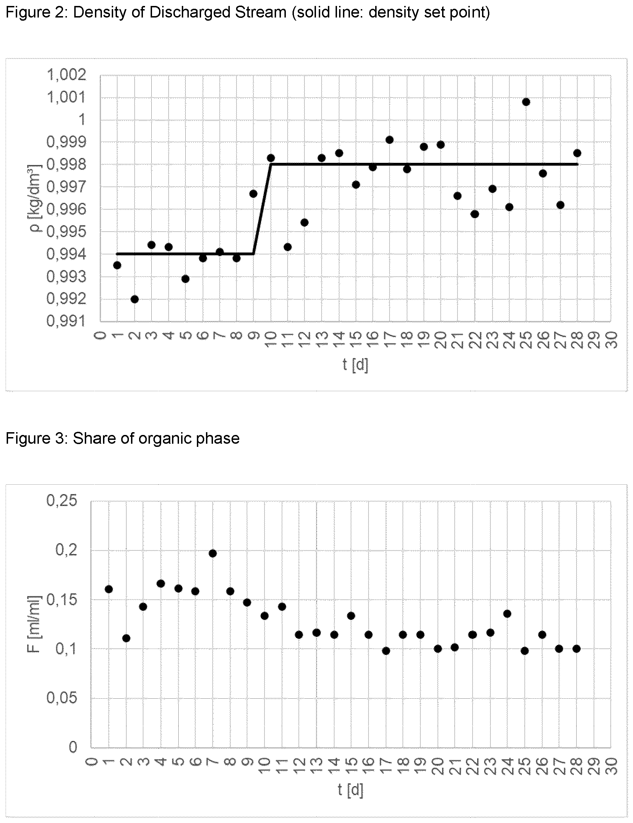

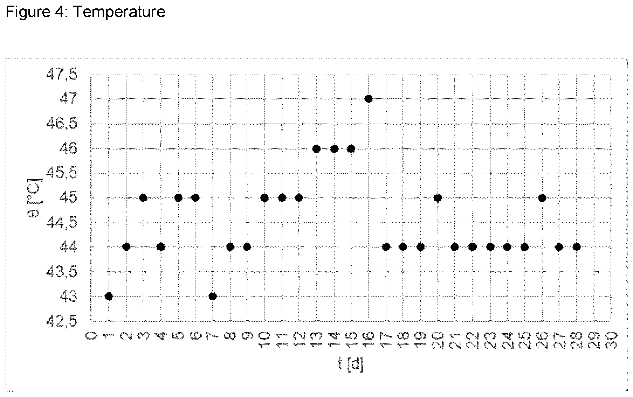

Analyses were recorded daily. The amounts of organic and aqueous phases were determined by collecting the stream discharged at the bottom of the phase separator and measuring the volume of the individual phases after a settling period long enough to obtain two well separate and clear phases. Table 1 shows the results of this experiment. Figures 2, 3 and 4 show measured density and density set point, the share of the organic phase in the discharged mixture and the temperature of the discharged mixture, respectively, from Table 1.

This example shows that the density-controlled discharge of the aqueous phase allows to withdraw a mixture of a desired ratio of the two phases and thereby remove the aqueous phase completely without removing an undesirably high amount of organic phase, even where no temperature difference between a relatively hot reaction zone and a relatively cool aqueous phase settling zone can be determined. Since the density of the cobalt salt solution is above 1 kg/dm³, there is enough room for higher density set points, and analyses can be made continuously in a commercial plant, allowing faster control feedback, the amount of organic phase withdrawn from a commercial reactor can be even lower compared to the miniplant.

**Table 1**

| Day | Temperature [°C] | Density [kg/dm³] | Density Set Point [kg/dm³] | Aqueous phase [ml/h] | Organic phase [ml/h] | Share of organic phase [ml/ml] |
|---|---|---|---|---|---|---|
| 1 | 43 | 0,9935 | 0,994 | 47 | 9 | 0,16 |
| 2 | 44 | 0,992 | 0,994 | 56 | 7 | 0,11 |
| 3 | 45 | 0,994 | 0,994 | 54 | 9 | 0,14 |
| 4 | 44 | 0,994 | 0,994 | 55 | 11 | 0,17 |
| 5 | 45 | 0,993 | 0,994 | 52 | 10 | 0,16 |
| 6 | 45 | 0,994 | 0,994 | 53 | 10 | 0,16 |
| 7 | 43 | 0,994 | 0,994 | 53 | 13 | 0,20 |
| 8 | 44 | 0,994 | 0,994 | 53 | 10 | 0,16 |
| 9 | 44 | 0,997 | 0,994 | 52 | 9 | 0,15 |
| 10 | 45 | 0,998 | 0,998 | 52 | 8 | 0,13 |
| 11 | 45 | 0,994 | 0,998 | 54 | 9 | 0,14 |
| 12 | 45 | 0,995 | 0,998 | 54 | 7 | 0,11 |
| 13 | 46 | 0,998 | 0,998 | 53 | 7 | 0,11 |
| 14 | 46 | 0,999 | 0,998 | 54 | 7 | 0,11 |
| 15 | 46 | 0,997 | 0,998 | 52 | 8 | 0,13 |
| 16 | 47 | 0,998 | 0,998 | 54 | 7 | 0,11 |
| 17 | 44 | 0,999 | 0,998 | 55 | 6 | 0,10 |
| 18 | 44 | 0,998 | 0,998 | 54 | 7 | 0,11 |
| 19 | 44 | 0,999 | 0,998 | 54 | 7 | 0,11 |
| 20 | 45 | 0,999 | 0,998 | 54 | 6 | 0,1 |
| 21 | 44 | 0,997 | 0,998 | 53 | 6 | 0,10 |
| 22 | 44 | 0,996 | 0,998 | 54 | 7 | 0,11 |
| 23 | 44 | 0,997 | 0,998 | 53 | 7 | 0,12 |
| 24 | 44 | 0,996 | 0,998 | 51 | 8 | 0,14 |
| 25 | 44 | 1,000 | 0,998 | 55 | 6 | 0,10 |
| 26 | 45 | 0,998 | 0,998 | 54 | 7 | 0,11 |
| 27 | 44 | 0,996 | 0,998 | 54 | 6 | 0,10 |
| 28 | 44 | 0,999 | 0,998 | 54 | 6 | 0,10 |

## Claims

1. Process for hydroformylation of olefins having 6 to 20 carbon atoms in the presence of a cobalt catalyst in the presence of an aqueous phase with thorough mixing in a reactor wherein a hydroformylation products-containing first stream is withdrawn at the top of the reactor and an aqueous phase-containing second stream is withdrawn from the bottom of the reactor via at least one line leading out of the bottom of the reactor, which process comprises controlling one or more mass flow parameters of the second stream in accordance with the density of the second stream.

2. Process according to claim 1, wherein the density of the second stream is measured in the line leading out of the bottom of the reactor.

3. Process according to claim 1 or claim 2, wherein one or more mass flow parameters are the mass flow, the mass flow rate or any other parameter determined on the basis of the mass flow and/or the mass flow rate.

4. Process according to any of the claims 1 to 3, comprising measuring the density of the second stream to determine one or more controlled variables on basis of the measured density, comparing the one or more controlled variables with one or more reference values to determine one or more controlling quantities and using the one or more controlling quantities to determine one or more actuating signals to control the one or more mass flow parameters of the second stream.

5. Process according to any of the claims 1 to 4, wherein the density is measured by one or more Coriolis mass flow meters.

6. Process according to any of the claims 1 to 5, wherein the measured density of the second stream is compared with a predefined reference value for the density of the second stream and the mass flow and/or the mass flow rate of the second is increased if the measured density of the second stream is above the predefined reference value for the density of the second stream.

7. Process according to any of the claims 1 to 6, wherein the measured density of the second stream is compared with a predefined reference value for the density of the second stream and the mass flow and/or the mass flow rate of the second stream is decreased if the measured density of the second stream is below the predefined reference value for the density of the second stream.

8. Process according to any of the claims 1 to 7, wherein the density of the second stream is measured at at least one point in the line leading out of the bottom of the reactor.

9. Process according to any of the claims 1 to 8, wherein the first stream and the second stream are passed into a post-reactor.

10. Process according to claim 9, wherein a hydroformylation products containing first stream is withdrawn at the top of the post-reactor and an aqueous phase-containing second stream is withdrawn from the bottom of the post-reactor.

11. Process according to claim 10, wherein the one or more mass flow parameters of the second stream withdrawn from the bottom of the post-reactor via at least one line leading out of the bottom of the post-reactor is controlled in accordance with the density of the second stream.

12. Process according to any of the claims 1 to 11, wherein the first stream and second stream, withdrawn from the reactor or in case a post-reactor is in place withdrawn from the post-reactor, are subjected in the presence of aqueous cobalt(ll) salt solution to oxygen treatment wherein the cobalt catalyst decomposes to form cobalt(II) salts which are extracted into the aqueous phase and the phases are then separated.

13. Process according to claim 12, wherein the water content in the reactor, in case a post-reactor is used in the post-reactor and in the phase separation is controlled by means of dynamic decoupling.

14. Process according to any of the claims 1 to 13, wherein the temperature in the reactor is from 100 to 250 °C and in case a post-reactor is used, the temperature in the post-reactor is from 100 to 250° C.

15. Process according to any of the claims 1 to 14, wherein the prevailing pressure in the reactor is from 100 to 400 bar abs and in case a post-reactor is used, the prevailing pressure in the post-reactor is from 100 to 400 bar abs.

## Patentansprüche

1. Prozess zur Hydroformylierung von Olefinen mit 6 bis 20 Kohlenstoff-Atomen in der Gegenwart eines Kobalt-Katalysators in der Gegenwart einer wässrigen Phase mit gründlichem Mischen in einem Reaktor, wobei ein Hydroformylierungsprodukte enthaltender erster Strom oben am Reaktor entnommen wird und ein wässrige Phase enthaltender zweiter Strom unten am Reaktor über mindestens eine Leitung entnommen wird, die aus dem Boden des Reaktors herausführt, wobei der Prozess Regeln von mindestens einem Massestromparameter des zweiten Stroms gemäß der Dichte des zweiten Stroms umfasst.

2. Prozess nach Anspruch 1, wobei die Dichte des zweiten Stroms in der Leitung gemessen wird, die aus dem Boden des Reaktors herausführt.

3. Prozess nach Anspruch 1 oder 2, wobei mindestens ein Massestromparameter der Massestrom, die Massestromrate oder jeder andere Parameter ist, der auf der Grundlage des Massestroms und/oder der Massestromrate bestimmt wird.

4. Prozess nach einem der Ansprüche 1 bis 3, Messen der Dichte des zweiten Stroms, um mindestens eine geregelte Variable auf der Grundlage der gemessenen Dichte zu bestimmen, Vergleichen der mindestens einen geregelten Variablen mit mindestens einem Referenzwert, um mindestens eine Regelungsquantität zu bestimmen, und Verwenden der mindestens einen Regelungsquantität umfassend, um mindestens ein Betätigungssignal zu bestimmen, um den mindestens einen Massestromparameter des zweiten Stroms zu regeln.

5. Prozess nach einem der Ansprüche 1 bis 4, wobei die Dichte durch mindestens einen Coriolis-Massestrommesser gemessen wird.

6. Prozess nach einem der Ansprüche 1 bis 5, wobei die gemessene Dichte des zweiten Stroms mit einem vordefinierten Referenzwert für die Dichte des zweiten Stroms verglichen wird und der Massestrom und/oder die Massestromrate des zweiten erhöht wird, wenn die gemessene Dichte des zweiten Stroms über dem vordefinierten Referenzwert für die Dichte des zweiten Stroms liegt.

7. Prozess nach einem der Ansprüche 1 bis 6, wobei die gemessene Dichte des zweiten Stroms mit einem vordefinierten Referenzwert für die Dichte des zweiten Stroms verglichen wird und der Massestrom und/oder die Massestromrate des zweiten Stroms vermindert wird, wenn die gemessene Dichte des zweiten Stroms unter dem vordefinierten Referenzwert für die Dichte des zweiten Stroms liegt.

8. Prozess nach einem der Ansprüche 1 bis 7, wobei die Dichte des zweiten Stroms an mindestens einem Punkt in der Leitung gemessen wird, die aus dem Boden des Reaktors herausführt.

9. Prozess nach einem der Ansprüche 1 bis 8, wobei der erste Strom und der zweite Strom in einen Nachreaktor überführt werden.

10. Prozess nach Anspruch 9, wobei ein Hydroformylierungsprodukte enthaltender erster Strom oben aus dem Nachreaktor entnommen wird und ein wässrige Phase enthaltender zweiter Strom unten aus dem Nachreaktor entnommen wird.

11. Prozess nach Anspruch 10, wobei der mindestens eine Massestromparameter des zweiten Stroms, der unten aus dem Nachreaktor über mindestens eine Leitung entnommen wird, die aus dem Boden des Nachreaktors herausführt, gemäß der Dichte des zweiten Stroms geregelt wird.

12. Prozess nach einem der Ansprüche 1 bis 11, wobei der erste Strom und der zweite Strom, die aus dem Reaktor oder, in dem Fall, dass ein Nachreaktor vorhanden ist, aus dem Nachreaktor entnommen werden, in der Gegenwart einer wässrigen Kobalt(II)-Salzlösung einer Sauerstoff-Behandlung unterworfen werden, wobei der Kobalt-Katalysator zersetzt wird, um Kobalt(II)-Salze auszubilden, die in die wässrige Phase extrahiert werden, und die Phasen dann separiert werden.

13. Prozess nach Anspruch 12, wobei der Wassergehalt in dem Reaktor, in dem Fall, dass ein Nachreaktor verwendet wird, in dem Nachreaktor, und in der Phasentrennung mittels dynamischem Entkoppeln geregelt wird.

14. Prozess nach einem der Ansprüche 1 bis 13, wobei die Temperatur in dem Reaktor zwischen 100 und 250 °C liegt und in dem Fall, dass ein Nachreaktor verwendet wird, die Temperatur in dem Nachreaktor zwischen 100 und 250 °C liegt.

15. Prozess nach einem der Ansprüche 1 bis 14, wobei der in dem Reaktor vorherrschende Druck zwischen 100 und 400 bar absolut liegt und, in dem Fall, dass ein Nachreaktor verwendet wird, der in dem Nachreaktor vorherrschende Druck zwischen 100 und 400 bar absolut liegt.

## Revendications

1. Procédé pour l'hydroformylation d'oléfines ayant 6 à 20 atomes de carbone en la présence d'un catalyseur au cobalt en la présence d'une phase aqueuse avec un mélange minutieux dans un réacteur, un premier flux contenant des produits d'hydroformylation étant soutiré au niveau de la partie supérieure du réacteur et un deuxième flux contenant une phase aqueuse étant soutiré de la partie inférieure du réacteur via au moins une ligne conduisant à l'extérieur de la partie inférieure du réacteur, lequel procédé comprend la régulation d'un ou plusieurs paramètres de flux de masse du deuxième flux conformément à la densité du deuxième flux.

2. Procédé selon la revendication 1, la densité du deuxième flux étant mesurée dans la ligne conduisant à l'extérieur de la partie inférieure du réacteur.

3. Procédé selon la revendication 1 ou la revendication 2, un ou plusieurs paramètres de flux de masse étant le flux de masse, le débit massique ou un quelconque autre paramètre déterminé sur la base du flux de masse et/ou du débit massique.

4. Procédé selon l'une quelconque des revendications 1 à 3, comprenant la mesure de la densité du deuxième flux pour déterminer une ou plusieurs variables régulées sur la base de la densité mesurée, la comparaison de la ou des variables régulées avec une ou plusieurs valeurs de référence pour déterminer une ou plusieurs quantités de régulation et l'utilisation de la ou des quantités de régulation pour déterminer un ou plusieurs signaux d'actionnement pour réguler le ou les paramètres de flux de masse du deuxième flux.

5. Procédé selon l'une quelconque des revendications 1 à 4, la densité étant mesurée par un ou plusieurs débitmètres massiques de Coriolis.

6. Procédé selon l'une quelconque des revendications 1 à 5, la densité mesurée du deuxième flux étant comparée à une valeur de référence prédéfinie pour la densité du deuxième flux et le flux de masse et/ou le débit massique du deuxième étant augmentés si la densité mesurée du deuxième flux est supérieure à la valeur de référence prédéfinie pour la densité du deuxième flux.

7. Procédé selon l'une quelconque des revendications 1 à 6, la densité mesurée du deuxième flux étant comparée à une valeur de référence prédéfinie pour la densité du deuxième flux et le flux de masse et/ou le débit massique du deuxième flux étant diminués si la densité mesurée du deuxième flux est inférieure à la valeur de référence prédéfinie pour la densité du deuxième flux.

8. Procédé selon l'une quelconque des revendications 1 à 7, la densité du deuxième flux étant mesurée au niveau d'au moins un point dans la ligne conduisant à l'extérieur de la partie inférieure du réacteur.

9. Procédé selon l'une quelconque des revendications 1 à 8, le premier flux et le deuxième flux étant passés dans un post-réacteur.

10. Procédé selon la revendication 9, un premier flux contenant des produits d'hydroformylation étant soutiré au niveau de la partie supérieure du post-réacteur et un deuxième flux contenant une phase aqueuse étant soutiré de la partie inférieure du post-réacteur.

11. Procédé selon la revendication 10, le ou les paramètres de flux de masse du deuxième flux soutiré de la partie inférieure du post-réacteur via au moins une ligne conduisant à l'extérieur de la partie inférieure du post-réacteur étant régulés conformément à la densité du deuxième flux.

12. Procédé selon l'une quelconque des revendications 1 à 11, le premier flux et le deuxième flux, soutirés du réacteur ou, dans le cas où un post-réacteur est en place, soutirés du post-réacteur, étant soumis, en la présence d'une solution aqueuse de sel de cobalt (II), à un traitement à l'oxygène dans lequel le catalyseur au cobalt se décompose pour former des sels de cobalt (II) qui sont extraits dans la phase aqueuse et les phases étant ensuite séparées.

13. Procédé selon la revendication 12, la teneur en eau dans le réacteur, dans le cas où un post-réacteur est utilisé, dans le post-réacteur et dans la séparation de phase étant régulée au moyen d'un découplage dynamique.

14. Procédé selon l'une quelconque des revendications 1 à 13, la température dans le réacteur étant de 100 à 250 °C et, dans le cas où un post-réacteur est utilisé, la température dans le post-réacteur étant de 100 à 250 °C.

15. Procédé selon l'une quelconque des revendications 1 à 14, la pression régnant dans le réacteur étant de 100 à 400 bars abs et, dans le cas où un post-réacteur est utilisé, la pression régnant dans le post-réacteur étant de 100 à 400 bars abs.
